Europäisches Patentamt

European Patent Office

Office européen des brevets

(11) Publication number: **0 080 536**
**A1**

(12) # EUROPEAN PATENT APPLICATION

(21) Application number: 81201320.9

(22) Date of filing: 02.12.81

(51) Int. Cl.³: **A 61 N 1/36**

(43) Date of publication of application:
08.06.83 Bulletin 83/23

(84) Designated Contracting States:
AT BE CH DE FR GB IT LI LU NL SE

(71) Applicant: Spaa, Walter
Trompstraat 71
NL-2518 BM The Hague(NL)

(72) Inventor: Spaa, Walter
Trompstraat 71
NL-2518 BM The Hague(NL)

(54) Cardiac stimulator.

(57) A cardiac pacemaker comprising a combination of high voltage pulse generating means and a high ohmic current-carrying member for supplying electrical current to an area of a body to be stimulated.

In a preferred embodiment the current-carrying member includes a conventional catheter containing a conventional liquid solution, such as a saline solution, which provides a path for conducting electrical current pulses to the area to be stimulated.

The invention relates to a cardiac stimulator (pacemaker). In particular, the invention relates to a cardiac stimulator adapted either to a) stimulate the heart or another organ or both these organs of a patient by means of electrical current, especially one or more electrical pulses, in an emergency situation arising, for example, during (intensive) care of this patient, or b) to temporarily vary the heart rhythm of a patient for diagnostic purposes.

Normally, when a patient is in (intensive) care, use is made of a catheter by means of which it is possible only to transfer measuring signals, which are indicative of parameters characteristic of a physical condition of the patient, from this patient to external measuring or monitoring equipment. In the event of an unexpected emergency situation requiring the heart and/or another organ to be stimulated, a stimulating catheter must be inserted as rapidly as possible. This generally used method is not only time-wasting and cumbersome, which can have fatal consequences in an emergency situation, but also means additional risk and/or inconvenience for the patient. These objections likewise apply to the situation in which the heart rhythm is to be temporarily varied, although then the requirement of rapid insertion is less important.

The above objections may be met by using a catheter of special structure employing electrodes that are electrically connectable through metallic conductors extending in the catheter to external equipment, thus permitting the derivation of signals from the inside the heart and/or the stimulation of the heart by means of electrical pulses.

Such special catheters, however, are relatively expensive. From considerations of economy and on the basis of statistical data, it is therefore considered desirable and justifiable to normally use catheters of simpler and cheaper structure in (intensive) care.

It is an object of the present invention to eliminate the above drawbacks and render it possible to electrically stimulate the heart and/or other organs of a patient in a simple and rapid manner while using catheters of relatively simple and cheap structure.

The present invention is based on the idea of using a non-metallic medium of low electrical conductivity for the transport of the electrical current required for cardiac stimulation, which medium is included in a catheter of normal and relatively cheap structure. In particular, for the purpose of such transport of current use may be made of either the column of liquid present in the catheter, particularly an electrolytic solution, or a thin non-metallic film of low electrical conductivity provided on the inner wall of the catheter, or fibrous cores of a low electrical conductivity material extending in a wall of such a catheter.

A cardiac stimulator according to the invention is characterized by an electrical high voltage circuit adapted to produce at its output such a high voltage that in a high impedance load circuit electrically connected to this output an electrical stimulus of an intensity and waveform suitable for cardiac stimulation is produced.

In particular, a cardiac stimulator according to the invention is characterized in that the aforesaid output is adapted to be connected to a lumen of a catheter so that this output is electrically connected to either an electrolytic solution present in this lumen, or a thin non-metallic film of low electrical conductivity provided on the inner wall of the lumen, or one or more fibrous cores of a low electrical conductivity material extending in the wall of the lumen.

A preferred embodiment of a cardiac stimulator according to the invention is characterized by a known per se cardiac stimulator generator including facilities permitting an optional adjustment of the signal produced by this generator at its output; the aforesaid electrical high voltage circuit includes a control input connected to the output of the cardiac stimulator generator.

The invention will be described in greater detail hereinafter.

A cardiac stimulator according to the invention is capable of performing all of the activities feasible with conventional cardiac stimulators, but using a catheter that need not include metallic conductors extending therein and stimulation electrodes connected therewith.

In other words, a cardiac stimulator according to the invention is adapted to transport the electrical signals required for a desired form of cardiac stimulation via a non-metallic current path of low electrical conductivity, i.e. high electrical impedance.

The aforesaid activities are, inter alia:

a) the application of fixed frequency stimulation pulses;

b) the application of stimulation pulses only in response to a request signal produced by a detector only in the event of an atrium or a ventricle or both skipping a beat;

c) a combination of the activities sub a) and b);

d) the application of stimulation pulses of a frequency higher than the normal heart rhythm, if necessary in combination with facilities for automatically switching to a desired rhythm;

e) the defibrillation of an atrium and/or ventricle by the application of one or more pulses having suitable characteristics, if necessary in combination with facilities for measuring the changes resulting from such an activity, and the repetition of this procedure either on its own initiative or in response to an external command, if necessary in combination with the possibility to alter the characteristics of the respective pulse or pulses, until the desired result has been achieved;

f) cardioversion by the application of one or more pulses (so-called "burst-pacing"), which procedure may be repeated, automatically or not, until the desired effect has been reached:

g) the (temporary) application of signals for causing rhythm failures, for example for halting "re-entry" tachycardias;

h) the stimulation via the left/right ventricle or the left/right atrium, for example in emergencies during catheterization; and

i) the so-called "double-demand pacing".

A cardiac stimulator according to the invention comprises an electrical high voltage circuit adapted to produce electrical pulses of such intensity and waveform that, when such a high voltage circuit is electrically connected to a high impedance as presented by a non-metallic current path as formed by a catheter including no metallic conductors, the electrical current required for the desired operation is active at the desired place. The output of such an electrical high voltage generator includes a set of electrical supply leads electrically connected, on the one hand, to one or more electrodes to be placed on the patient and, on the other hand, to a metal valve, which may be of standard design, or an electrically conductive auxiliary means which for instance is adapted to be mounted on or to perforate the respective catheter. Such a valve or auxiliary means is arranged for forming an electrical connection with either an electrolytic solution present in the catheter, or a thin non-metallic film of low electrical conductivity provided on the inner wall of a lumen of the catheter, or one or more fibrous cores of a low electrical conductivity material extending in the wall of a lumen of this catheter.

In addition to unipolar operation, in which only one liquid column of an electrolytic solution or one thin non-metallic film on the inner wall or one set of fibrous cores is required, also either bipolar or multipolar operation falls within the scope of the present invention. In that case, however, two or more separate liquid columns of an electrolytic solution or two or more separate thin non-metallic films on the inner wall or two or more separate sets of fibrous cores of a low electrical conductivity material are required.

An electrical high voltage circuit according to the invention may be combined with facilities suited to prevent, inter alia, firing at inappropriate moments.

A preferred embodiment of a cardiac stimulator according to the invention includes a cardiac stimulator generator of known per se arrangement with facilities permitting an optional adjustment of the signal produced by this generator at its output. The electrical high voltage circuit in this preferred embodiment includes a control input connected to the output of this known per se cardiac stimulator generator.

In a preferred embodiment of the invention an electrical generator for causing the muscles of a body to react to an electrical stimulus is adapted to cause an electrical current in the range of f.i. 1-5 milliamps to flow through an electrically conductive liquid, for example a conventional saline solution, providing a current path to the area of the body to be stimulated. In particular, an electric generator of this type compromises an output circuit for producing a high voltage, for example in the range of 1-10 kilovolts, across an electrical load including this electrically conductive liquid with an internal impedance of, for example, $10^6$ ohms looking into the output circuit of the electrical generator, and an overall impedance of $10^6$

ohms for the electrical load, a voltage of 10 kilovolts produced at the output of the generator across the load causes a current of 5 milliamps to flow therethrough. In a simple embodiment the output circuit includes a transformer adapted to produce across its output-winding the high voltage as required for causing the relatively low current to flow through the load.

In another embodiment such a cardiac stimulator according to the invention comprises an electrical high voltage circuit, consisting of a high voltage generator which forms together with or without passive or active components a high voltage circuit adapted to produce electrical pulses of such an intensity, waveform and repetition rate that, when such a high voltage circuit is connected with a catheter which is part of the load and having an impedance ranging from a few Ohms up to some Mega Ohms, an electrical current is generated for the desired operation, active at the desired place.

Such a circuit arrangement for instance comprises a feedback control circuit which is adapted for maintaining the output current within the desired limits.

In another embodyment said circuitarrangement includes measuring means for measuring the loadresistance and adjusting, in response to the measuring results, the high voltage in order to achieve the desired output-current.

The invention is not limited to such embodiments.

I CLAIM

1. A cardiac stimulator, characterized by an electrical high voltage circuit adapted to produce at its output such a high electrical voltage that in a high impedance load circuit electrically connected to said output an electrical stimulus of an intensity and waveform suitable for cardiac stimulation is produced.

2. A cardiac stimulator according to claim 1, characterized in that said output is adapted to be connected to a current path of said load circuit, said current path including an electrolytic solution.

3. A cardiac stimulator according to claim 1, characterized in that said output is adapted to be connected to a current path of said load circuit, said current path includes a thin non-metallic film.

4. A cardiac stimulator according to claim 1, characterized in that said output is adapted to be connected to one or more fibrous non-metallic current paths of said load circuit.

5. A cardiac stimulator according to claim 1 or 2, characterized in that said output is adapted to be connected to a lumen of a catheter so that said output is electrically connected to an electrolytic solution present in said lumen.

6. A cardiac stimulator according to claim 1 or 3, characterized in that said output is adapted to be connected to the inner wall of a lumen of a catheter so that said output is electrically connected to a film of low electrical conductivity material provided on said inner wall.

7. A cardiac stimulator according to claim 1 or 4, characterized in that said output is adapted to be connected to a wall of a lumen of a catheter so that said output is electrically connected to one or more fibrous cores of a low electrical conductivity material extending in said wall.

8. A cardiac stimulator according to any one of the preceding claims, characterized by a known per se cardiac stimulator generator including facilities permitting an optional adjustment of the signal produced by said generator at its output; said electrical high voltage circuit including a control input connected to the output of said cardiac stimulator generator.

9. A cardiac stimulator according to any one of the preceding claims characterized by an electrical high voltage circuit adapted to produce at its output such a voltage that in a load of any impedance ranging from a few Ohms to some Mega Ohms, connected to said output, an electrical current of an intensity, waveform and repetitionrate as suited for stimulation is produced.

10. A cardiac stimulator according to any one of the preceding claims, characterized in that said output is protected against generation of electrical pulses with such a energy content and or repetitionrate that undesired situations could occur.

**European Patent Office**

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | EP-A-0 029 479 (TRANSFORMATOREN-UND RONTGENWERK) *Page 5, lines 20-31; page 6, lines 10-16* | 1,9,10 | A 61 N 1/36 |
| A | US-A-3 554 198 (TATOIAN) *Column 2, lines 10-13 and 51-61* | 1,8 | |
| A | US-A-3 109 430 (TISCHLER) *Column 1, lines 28-33; column 2, lines 3-7 and 18-22* | 1,8 | |
| A | FR-A-1 557 087 (THOMSON) *Page 1, right-hand column, lines 21-27; page 2, right-hand column, paragraphs 2,5 and 6* | 1,3,4, 6,7 | |
| A | DE-B-1 290 962 (TELEFUNKEN) *Column 1, line 63 to column 2, line 2; column 4, lines 28 and 33* | 1 | TECHNICAL FIELDS SEARCHED (Int. Cl. 3) A 61 N |
| A | FR-A-1 571 849 (TELEFUNKEN) *Page 2, lines 1-10* & GB - A - 1 179 090 | 1,2,5 | |

.../.

The present search report has been drawn up for all claims

| Place of search THE HAGUE | Date of completion of the search 30-06-1982 | Examiner SIMON J.J.E. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent
Office

**EUROPEAN SEARCH REPORT**

| | DOCUMENTS CONSIDERED TO BE RELEVANT | | Page   2 |
|---|---|---|---|

| Category | Citation of document with indication, where appropriate, of relevant passages | Relevant to claim | CLASSIFICATION OF THE APPLICATION (Int. Cl. ³) |
|---|---|---|---|
| A | GB-A-1 536 270  (GREEN) <br> *Page  2,  lines  26-50,66-69 and 109-112; page 2, line 125 to page 3, line 3* | 2,5,6 | |
| | --- | | |
| A | DE-A-2 810 004  (NEEDLE) <br> *Page 5, paragraphs 2 and 3; page 7, first paragraph; page 8, lines 20 and 21* | 4,7 | |
| | ----- | | |
| | | | TECHNICAL FIELDS SEARCHED (Int. Cl. ³) |

The present search report has been drawn up for all claims

| Place of search <br> THE HAGUE | Date of completion of the search <br> 30-06-1982 | Examiner <br> SIMON J.J.E. |
|---|---|---|

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO Form 1503 03 82